## Europäisches Patentamt

## European Patent Office

## Office européen des brevets

(11) Publication number: **0 064 555**
**B1**

## EUROPEAN PATENT SPECIFICATION

(45) Date of publication of patent specification: **24.04.85**

(21) Application number: **81903007.3**

(22) Date of filing: **06.11.81**

(86) International application number:
**PCT/JP81/00320**

(87) International publication number:
**WO 82/01707 27.05.82 Gazette 82/14**

(51) Int. Cl.⁴: **C 07 G 11/00,** C 12 P 1/06,
A 61 K 35/74

(54) **ANTIBIOTIC 5057B.**

(30) Priority: **13.11.80 JP 158856/80**

(43) Date of publication of application:
**17.11.82 Bulletin 82/46**

(45) Publication of the grant of the patent:
**24.04.85 Bulletin 85/17**

(84) Designated Contracting States:
**CH DE FR GB LI NL**

(56) References cited:
**Aida Hiroshi "Oyo Biseibutsu-gaku" (1979-3-1),
Dobun Shoin, P. 188 - 203
CHEMICAL ABSTRACTS, vol. 88, no. 19, 1978,
page 392, abstract no. 134856j, COLUMBUS,
OHIO (US)**

(73) Proprietor: **KAKEN PHARMACEUTICAL CO.,
LTD.**
**No. 28-8, 2-chome, Honkomagome Bunkyo-Ku
Tokyo 113 (JP)**

(72) Inventor: **KUSAKABE, Yoko**
**Kaken Chemical Central Laboratory
6-42 Jujodai 1-chome Kita-ku Tokyo 114 (JP)**
Inventor: **MIZUNO, Taku**
**Kaken Chemical Central Laboratory
6-42, Jujodai 1-chome Kita-ku Tokyo 114 (JP)**

(74) Representative: **Vossius Vossius Tauchner
Heunemann Rauh
Siebertstrasse 4 P.O. Box 86 07 67
D-8000 München 86 (DE)**

EP 0 064 555 B1

Courier Press, Leamington Spa, England.

## Description

The present invention relates to a new antibiotic which has been called 5057B and a process for its production.

The present inventors previously found that an actinomycete *Streptomyces sp. 5057* strain belonging to the genus *Streptomyces* (FERM BP-62) produces a substance having antibacterial activity and isolated the antibiotic 5057(A) from the culture broth; see Japanese Patent Publication No. 8641/1979 and "Chemical Abstracts", *88*: 134856j (1978).

The present inventors, in the course of further research, have now found that a new substance different from the antibiotic 5057A is present in said culture broth and isolated the same, which is designated antibiotic 5057B.

The antibiotic 5057B, in view of its physicochemical and biological properties, is an antibiotic belonging to the group of polyether antibiotics. Among the polyether antibiotics there are known, as those showing ultraviolet absorption in the vicinity of 290 nm, lysocellin (The Journal of Antibiotics, Vol. 28, pp. 118—121, 1975) and the antibiotic 5057A (Japanese Patent Publication No. 8641/1979). The antibiotic 5057B is a new compound which differs from the above-mentioned known antibiotics in the melting point, specific rotation, infrared absorption spectrum, and the Rf value on silica gel thin layer chromatography.

The present invention therefore relates to the new antibiotic 5057B having potent antibiotic activity, especially against gram-positive bacteria, and a process for its preparation comprising cultivating a microorganism belonging to the genus *Streptomyces* which has the ability to produce the antibiotic 5057B and then recovering the antibiotic 5057B from the culture broth.

The strain *Streptomyces sp. 5057*, belonging to the genus *Streptomyces*, preferably employed in the production of the antibiotic 5057B was deposited on March 4, 1976 and identically redeposited on May 1, 1981 (in accordance with Rule 28 EPC) with the Fermentation Research Institute, Agency of Industrial Science and Technology (No. 1—3, Yatabechohigashi 1-chome, Tsukuba District, Ibaragi Prefecture, Japan) and accorded the accession number FERM No. 3464 and FERM BP-62 respectively. The microbiological properties of the strain are described in detail in the specification of Japanese Patent Publication No. 8641/1979.

In order to obtain the antiobiotic 5057B according to the present invention, ordinary methods for cultivation of actinomycetes can be employed, but a cultivation with aeration and agitation is suitable for industrial production. The cultivation temperature of 25—35°C is usual, but the temperature of 30°C is preferred. As a culture medium, there can be used one customarily employed for cultivation of microorganisms belonging to the genus *Streptomyces*, for example one containing carbon sources such as glucose, starch, glycerol, dextrin, sucrose, and animal or vegetable oils and nitrogen sources such as soybean meal, corn steep liquor, wheat germs and ammonia. Further, if necessary, inorganic salts such as calcium carbonate, sodium chloride, potassium chloride, and phosphates can be added; it is also possible to add organic or inorganic salts having an action to assist the growth of the microorganism and stimulate the production of the antibiotic 5057B. The accumulation of the antibiotic 5057B in the culture medium reaches a maximum usually 4 to 8 days after the start of the cultivation in both shaking and tank cultures.

The antibiotic 5057B is recovered from the culture broth by utilizing its physicochemical properties; since the substance is lipo-soluble and acidic, there can be used, in a suitable combination, extractions with a variety of organic solvents, chromatography on a variety of activated adsorbents, and other methods. For example, an isolation method consisting of a combination of extraction and chromatography is carried out as follows: the culture broth, after addition of a filter aid such as diatomaceous earth or Radiolite® 700 is filtered. The filtrate and the microbial cells are each extracted with a suitable solvent such as ethyl acetate or acetone. The cell extract and the filtrate extract are combined. Evaporation of the solvent from the combined extracts leaves, as the residue, crude crystals of a mixture of the antibiotic 5057B and 5057A. The mixture is subjected to, for example, chromatography to isolate the antibiotic 5057B from the mixture. The eluate is concentrated under reduced pressure. Then the residue is dissolved in a suitable solvent, for example ethyl acetate, and treated first with a dilute hydrochloric acid solution and then with a dilute sodium carbonate solution. After concentrating the ethyl acetate solution under reduced pressure, crystals of sodium salt of the antibiotic 5057B sodium salt are formed. Recrystallization of the crystals from a suitable solvent such as n-hexane-ethyl acetate gives the pure sodium salt of the antibiotic 5057B. The antibiotic 5057B itself is acidic and its sodium salt has the following properties:

(1) Colorless needles
(2) Melting point: 143—145°C
(3) Elementary analysis (found %):

| C | H | O | Na |
|---|---|---|----|
| 59.96 | 9.03 | 27.60 | 3.41 |

(4) Specific rotation:
$[\alpha]_D^{25} = +5.5°$ (Cl, $CHCl_3$)

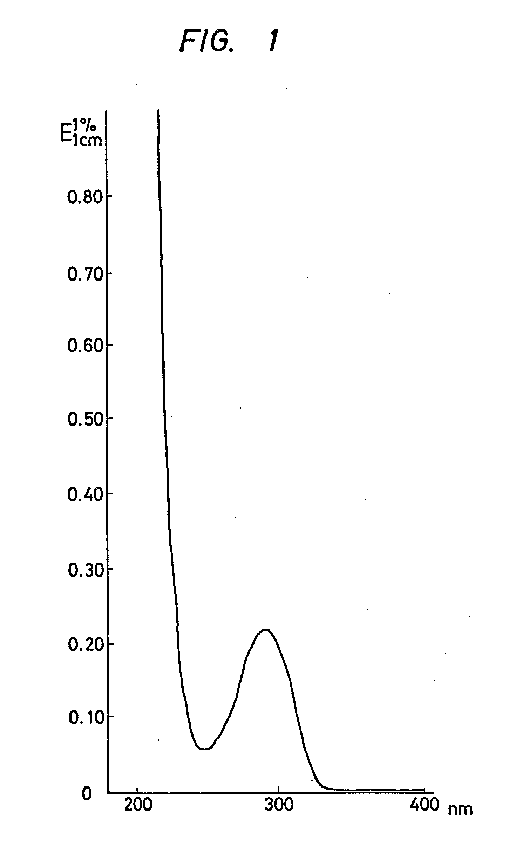

(5) Ultraviolet absorption spectrum:
The absorption spectrum taken in a 0.25% methanol solution is shown in Figure 1.
Maximum absorption:
$\lambda_{max}^{MeOH}$ $(E_{1\ cm}^{1\%})$ 290 nm (0.88)

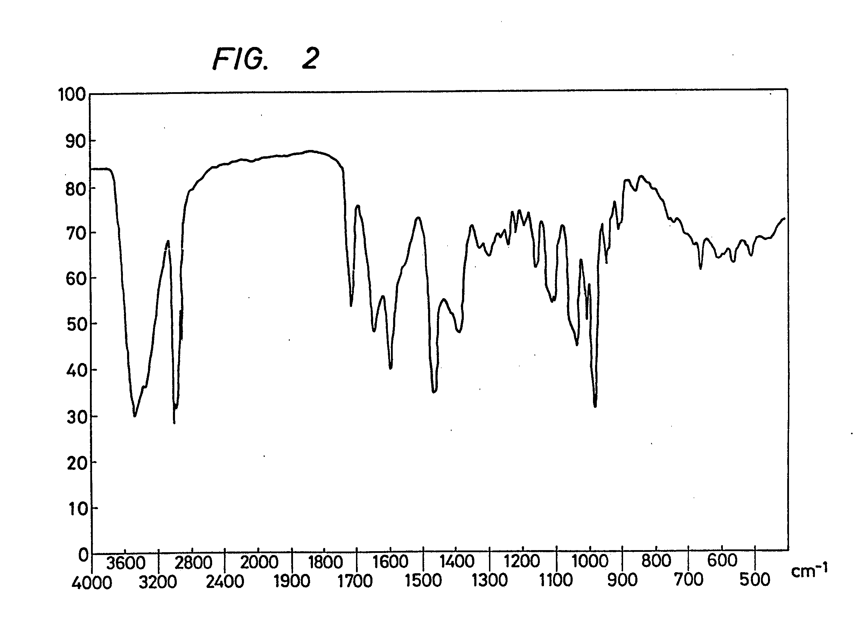

(6) Characteristic absorption $(cm^{-1})$:
In infrared absorption spectrum (taken in potassium bromide):
3480, 3000, 2970, 2812, 1719, 1645, 1595, 1465,
1385, 1160, 1100, 1035, 980
The infrared absorption spectrum is shown in Figure 2.

(7) Solubility:
Soluble in methanol, ethanol, ethyl acetate, chloroform, ether, acetone, and benzene, etc; insoluble in water.

(8) Color reaction:
Positive 2,4-dinitrophenylhydrazine reaction; negative ninhydrin reaction and vanilline-sulfuric acid reaction; colors with $I_2$ gas.

The sodium salt of the 5057B antibiotic has also the following additional properties:

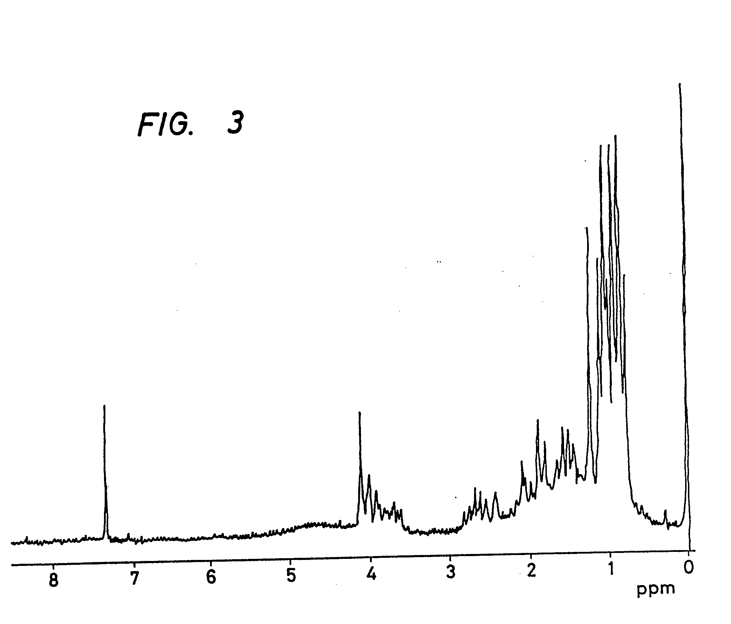

(9) Nuclear magnetic resonance spectrum (taken in $CDCl_3$)
Proton NMR spectrum is shown in Figure 3.

(10) Thin layer chromatography on silica gel: Kieselgel $GF_{254}$ manufactured by Merck Co.

| Solvent system | Rf value |
| --- | --- |
| Chloroform-methanol (20:1) | 0.30 |
| Ethyl acetate-methanol (20:1) | 0.64 |
| N-hexane-ethyl acetate (1:2) | 0.62 |
| Benzene-ethyl acetate (1:1) | 0.37 |

(11) The antibacterial spectrum is shown in Table I.
In the Table, the letter a means heart infusion agar medium, the letter b Kirchner's semifluid agar medium, the letter c potato-sucrose agar medium, and the letter d Sabouraud's 3% glucose agar medium. As can be seen from the antibacterial spectrum, the antibiotic 5057B sodium salt exhibits potent antibacterial activity especially against gram-positive microorganisms.

(12) Acute toxicity test:
Acute toxicity tests were performed with mice. The $LD_{50}$ value is 50 mg/kg in the case of intraperitoneal administration and 500 mg/kg or more in the case of oral administration.

Melting points, specific rotations and Rf values on silica gel thin layer chromatography of the 5057A and 5057B sodium salts are shown in Table II.

| Antibiotic | TABLE II<br>5057A | 5057B |
| --- | --- | --- |
| Melting point | 133—135°C | 143—145°C |
| Specific rotation | +1.0 (Cl, MeOH) | +5.5 (Cl, $CHCl_3$) |
| Rf value (1) | 0.56 | 0.62 |
| Rf value (2) | 0.28 | 0.37 |

The solvent systems used for the silica gel chromatography were n-hexane-ethyl acetate (1:2) and benzene-ethyl acetate (1:1) in (1) and (2), respectively.

# 0 064 555

TABLE I

| Test microorganism | Minimum inhibitory concentration ($\mu g/ml$) | Medium |
|---|---|---|
| *Staphylococcus aureus* FDA 209 PJC-1 | 0.5 | a |
| *Staphylococcus aureus* resistant to penicillins, streptomycin, kanamycin, chloramphenicol, and tetracyclines | 0.5 | a |
| *Bacillus subtilis* ATCC-6636 | 0.5 | a |
| *Sarcina lutea* NIHJ | 2 | a |
| *Micrococcus luteus* ATCC-398 | 0.5 | a |
| *Mycobacterium smegmatis* ATCC-607 | 5 | a |
| *Mycobacterium tuberculosis* $H_{37}R_v$ | 10 | b |
| *Escherichia coli* NIHJ JC-2 | 100 | a |
| *Escherichia coli* resistant to streptomycin, kanamycin, chloramphenicol, and tetracyclines | 100 | a |
| *Klebsiella pneumoniae* GN-6445 | 100 | a |
| *Proteus vulgaris* GN-75 | 100 | a |
| *Pseudomonas aeruginosa* IFO-3756 | 100 | c |
| *Penicillium citrinum* ATCC-9849 | 100 | c |
| *Aspergillus fumigatus* NI-5561 | 100 | c |
| *Alternaria kikuchiana* CBS | 100 | c |
| *Ophiobolus miyabeanus* ITO | 50 | c |
| *Nocardia asteroides* | 5 | d |
| *Candida albicans* | 50 | d |

The antibiotic 5057B is useful as an antibacterial agent. The following example illustrates the invention:

Example

The strain *Streptomyces sp. 5057* (FERM BP-62) is inoculated into 1 l of an aqueous medium (pH 6.0) composed of soluble starch 6.0%, soybean meal 2.5%, beer yeast 0.5%, potassium primary phosphate 0.5%, ammonium sulfate 0.3%, and calcium carbonate 0.3%. Cultivation is carried out at 30°C for 48 hours. The culture broth is transferred into 100 l of a medium having the same composition as set forth above and cultivation is carried out with stirring under aeration in a 200 l tank at 30°C for 96 hours. The rate of aeration is 100 l/min at a stirring rate of 250 rpm.

The culture broth, after addition of a filter aid (Radiolite® 700: registered trademark of Showa Chemical Industry Co.) is filtered to separate the filtrate and cells. The filtrate is extracted with 40 l of ethyl acetate. The cells are extracted with 30 l of acetone. The acetone extract is concentrated under reduced pressure. The residue obtained is extracted with 20 l of ethyl acetate. The extract thus obtained is combined with the ethyl acetate extract of the filtrate and the mixture is concentrated under reduced pressure. The residue is adsorbed on a column packed with 200 g of activated alumina slurried with the aid of ethyl acetate. The eluant is a mixture of ethyl acetate-ethanol (1:1). Active fractions are pooled and concentrated

4

to dryness under reduced pressure. The crystalline residue is a mixture of the antibiotic 5057B and 5057A in crude form.

The mixture thus obtained is applied to a column packed with 500 g of silica gel slurried with the aid of n-hexane-ethyl acetate (1:2). The same solvent mixture is used as eluant. The fractions obtained are subjected to silica gel thin layer chromatography. A mixture of n-hexane-ethyl acetate (1:2) is used as developing solvent. Only those fractions containing the 5057B substance are collected and concentrated under reduced pressure. The 5057A substance is eluted after the 5057B substance has been eluted. The residue containing the antibiotic 5057B is dissolved in ethyl acetate and is shaken together with dilute hydrochloric acid and then with a dilute sodium carbonate solution. The ethyl acetate solution is then concentrated under reduced pressure. The crystalline residue formed is recrystallized from n-hexane-ethyl acetate to give 0.85 g colorless needles of sodium salt of the antibiotic 5057B melting at 143—145°C.

Figure 1 shows ultraviolet absorption spectrum of sodium salt of the 5057B substance taken in a 0.25% methanol solution, Figure 2 infrared absorption spectrum of sodium salt of the 5057B substance, and Figure 3 proton NMR spectrum of sodium salt of the 5057B substance.

## Claims

1. Antibiotic 5057B which is an acid and which, as its sodium salt, has the following physicochemical properties:

(1) Colorless needles

(2) Melting point: 143—145°C

(3) Elementary analysis (found %):

| C | H | O | Na |
|---|---|---|---|
| 59.96 | 9.03 | 27.60 | 3.41 |

(4) Specific rotation:
$[\alpha]_D^{25} = +5.5°$ (C l, $CHCl_3$)

(5) Maximum absorption in ultraviolet absorption spectrum (taken in a 0.25% methanol solution):
$\lambda_{max}^{MeOH}$ ($E_1^{1\%}{}_{cm}$) 290 nm (0.88)

(6) Characteristic infrared absorption spectrum (in potassium bromide):
3480, 3000, 2970, 2812, 1719, 1645, 1595, 1465, 1385, 1160, 1100, 1035, 980 cm$^{-1}$

(7) Solubility:
Soluble in methanol, ethanol, ethyl acetate, chloroform, ether, acetone, and benzene; insoluble in water.

(8) Color reaction:
Positive 2,4-dinitrophenylhydrazine reaction; negative ninhydrin reaction and vanilline-sulfuric acid reaction; colors with $I_2$ gas.

2. A process for the preparation of the antibiotic 5057B according to Claim 1 which comprises cultivating a microorganism belonging to the genus *Streptomyces* which has the ability to produce the antibiotic 5057B and then recovering the antibiotic 5057B from the culture broth.

## Patentansprüche

1. Antibioticum 5057B, welches eine Säure ist und welches, in Form seines Natriumsalzes, die folgenden physikochemischen Eigenschaften hat:

(1) Farblose Nadeln,

(2) Schmelzpunkt: 143 bis 145°C

(3) Elementaranalyse (gefunden %):

| C | H | O | Na |
|---|---|---|---|
| 59,96 | 9,03 | 27,60 | 3,41 |

(4) Spezifische Drehung:
$[\alpha]_D^{25} = +5,5°$ (C l, $CHCl_3$)

**0 064 555**

(5) Maximale Absorption im Ultraviolett-Absorptions-spektrum (aufgenommen in einer 0,25% Methanollösung):
$\lambda_{max}^{MeOH}$ ($E_1^{1\%}{}_{cm}$) 290 nm (0,88)

(6) Charakteristiches Infrarot-Absorptionsspektrum (in Kaliumbromid):
3480, 3000, 2970, 2812, 1719, 1645, 1595, 1465, 1385, 1160, 1100, 1035, 980 cm$^{-1}$

(7) Löslichkeit:    Löslich in Methanol, Äthanol, Essigsäureäthylester, Chloroform, Äther, Aceton und Benzol; unlöslich in Wasser,

(8) Farbreaktion:
Positive 2,4-Dinitrophenylhydrazinreaktion; negative Ninhydrinreaktion und Vanillin-Schwefel-säure-Reaktion; Verfärbung mit $J_2$-Gas.

2. Ein Verfahren zur Herstellung des Antibiotikums 5057B nach Anspruch 1, welches Züchten eines zur Gattung *Streptomyces* gehörenden Mikroorganismus, der zur Erzeugung des Antibiotikums 5057B in der Lage ist, und dann Gewinnung des Antibiotikums 5057B aus der Kulturbrühe umfaßt.

**Revendications**

1. Antibiotique 5057B qui est un acide et qui, sous forme de son sel de sodium, a les propriétés physicochimiques suivantes:

(1) Aiguilles incolores.

(2) Point de fusion: 143—145°C

(3) Composition élémentaire (trouvé %)

| C | H | O | Na |
|---|---|---|---|
| 59,96 | 9,03 | 27,60 | 3,41 |

(4) Pouvoir rotatoire:
$[\alpha]_D^{25}=+5,5°$ (CI, CHCl$_3$)

(5) Absorption maxima dans le spectre d'absorption ultraviolette (pris dans une solution à 0,25% dans du méthanol):
$\lambda_{max}^{MeOH}$ ($E_1^{1\%}{}_{cm}$) 290 nm (0,88)

(6) Spectre infrarouge caractéristique (dans du bromure de potassium):
3480, 3000, 2970, 2812, 1719, 1645, 1595, 1465, 1385, 1160, 1100, 1035, 980 cm$^{-1}$.

(7) Solubilité:
Soluble dans le méthanol, l'éthanol, l'acétate d'éthyle, le chloroforme, l'éther, l'acétone, et le benzène. Insoluble dans l'eau.

(8) Réactions colorées:
Réaction à la 2,4-dinitrophénylhydrazine positive; réaction à la ninhydrine et à la vanilline-acide sulfurique négative. Se colore avec $I_2$ gazeux.

2. Procédé de préparation de l'antiobiotique 5057B selon la revendication 1, qui consiste à cultiver un microorganisme appartenant au genre *Streptomyces* qui a la capacité de produire l'antibiotique 5057B, puis à récupérer l'antibiotique 5057B à partir du bouillon de culture.

6

FIG. 1

FIG. 2

0 064 555

FIG. 3